# EUROPÄISCHE PATENTSCHRIFT

(11) **EP 0 595 311 B1**
(45) Veröffentlichungstag und Bekanntmachung des Hinweises auf die Patenterteilung: **22.01.1997**
(21) Anmeldenummer: 93117472.6
(22) Anmeldetag: 28.10.1993
(51) Int. Cl.: A61K 31/44, A61K 31/485

(54) **Kombinationspräparat aus Flupirtin und Morphin zur Behandlung von Schmerzen und zur Vermeidung der Morphin-Abhängigkeit**
Pharmaceutical composition consisting of flupirtin and morphine for the treatment of pain and to avoid a morphine addiction
Médicament composé de flupirtin et morphine pour le traitement de douleur et pour éviter une dépendance au morphine

(30) Priorität: 30.10.1992 DE 4236752
(43) Veröffentlichungstag der Anmeldung: 04.05.1994
(73) Patentinhaber: ASTA Medica Aktiengesellschaft, D-01277 Dresden (DE)
(72) Erfinder: Nickel, Bernd, Dr., D-64367 Mühltal (DE); Lobisch, Michael, Dr., D-64372 Ober-Ramstadt (DE); Szelenyi, Stefan, Prof. Dr., D-90571 Schwaig (DE); Engel, Jürgen, Prof. Dr., D-63755 Alzenau (DE); Emig, Peter, Dr., D-61138 Niederdorffelden (DE); Pergande, Gabriela, Dr., D-63071 Offenbach (DE)

(56) Entgegenhaltungen:
- EP-A- 0 207 193
- REGIONAL ANESTHESIS Bd. 18, Nr. 4S , Juli 1993 Seite 4 B. NICKEL ET AL. 'Pharmacological activity of flupirtine in combination with morphine'
- PHARM. ZEITUNG Bd. 137, Nr. 35 , 27. August 1992 Seiten 24 - 32 I. JURNA ET AL. 'Analgetisch und muskelrelaxierend: Flupirtin'
- NEUROPSYCHOBIOLOGIE Bd. 20, Nr. 3 , 1988 Seiten 164 - 168 W. DIMPFEL ET AL. 'Dose- and time-dependent action of morphine, tramadol and flupirtine as studied by radioelectroencephalography in the freely behaving rat'
- ARZNEIM.-FORSCHUNG Bd. 35, Nr. 1 , 1985 Seiten 75 - 77 W. SCHEEF ET AL. 'Flupirtin bei Patienten mit karzinombedingten Schmerzzuständen'

## Beschreibung

Das aus Opium, dem eingetrockneten Milchsaft unreifer Mohnkapseln (Papaver somniferum), gewonnene Morphin ist seit seiner Isolierung durch Sertürner (1806) in Form des Hydrochlorids als Mittel gegen starke Schmerzen im Gebrauch. Bei häufigem und lang andauerndem Gebrauch dieses Analgetikums, beispielsweise bei Tumorpatienten, besteht die Gefahr der Ausbildung einer Sucht und Toleranzentwicklung (Morphinismus).

Aber auch die beim bestimmungsgemäßen Gebrauch zu beobachtenden Nebenwirkungen wie beispielsweise euphorische Wirkung, emetische Wirkung, spastische Obstipation und Erhöhung des Tonus der glatten Muskulatur reduzieren die therapeutische Anwendbarkeit von Morphium.
So hat es nicht an Bemühungen gefehlt, stark wirkende, aber nebenwirkungsarme Analgetika synthetisch darzustellen. Das partial synthetische Produkt Diamorphin (Heroin) ist zwar 10mal wirksamer als Morphin, ruft aber wesentlich leichter Sucht hervor. Pethidin ist ca. 5mal schwächer wirksam als Morphin und ist auch weniger spasmogen.

Auch Pentazocin und Buprenorphin unterliegen auf Grund ihres Suchtpotentials dem Betäubungsmittelrecht.

Tramadol ist nur etwa 1/10 - 1/5 so stark wirksam wie Morphin, dafür besitzt es bis jetzt noch kein bekanntgewordenes Suchtpotential.

Es besteht also immer noch ein großer Bedarf nach einer zuverlässig, auch bei starken Schmerzen gut wirksamen analgetischen Medikation mit wenig Nebenwirkungen, die aus sozialen Gründen kein Suchtpotential aufweisen sollte.

Um den Verbrauch an Analgetika zu senken, oder um die nicht immer ausreichende analgetische Wirkung zu verstärken, hat man den Weg der Kombination von Wirkstoffen beschritten.
Man versucht dabei, durch Kombination von ausgewählten Analgetika mit Morphin die Nebenwirkungen des Morphin weniger stark in Erscheinung treten zu lassen und die analgetische Wirkung zu verstärken.

Da Morphin keine entzündungshemmende Wirkung hat, kann man dieses Defizit in der Morphinwirkung durch Kombination von Morphin mit antiphlogistisch oder antipyretisch wirkenden Analgetika ausgleichen. So beschreiben Vergoni et al. (Life Sci., 50(16), Seite 135-138 (1992)) die potenzierenden Effekte von Pinacidil auf den analgetischen Effekt von Morphin.

Eine Kombination aus rektal verabreichten Indomethacin mit intravenös verabreichten Morphin wird von Segstro und Morley-Forster im Can. J. Anaesth. 38(5), 578-581 (1991) beschrieben.

Tierversuche, die die Potenzierung von analgetischen Effekten von Morphin und Clonidin an Ratten beschreiben, werden von Wilcox, Carlsson, Jochim und Jurna in Brain Res. 405(1), 84-93 (1987) dargestellt.

Alle Versuche haben zum Ziel, die analgetischen Wirkungen im Sinne einer synergistischen Wirkung zu verstärken, um die Dosis an Analgetikum beziehungsweise Antiphlogistikum und Morphin zu reduzieren.

Flupirtin (INN) ist ein Analgetikum mit muskelrelaxierender Wirkkomponente. (B. Nickel, V. Jakovlev, I. Szelenyi, Arzneim.-Forsch. 40(II)8, 909-911 (1990) DE-OS 36 01 195).

Es verfügt über kein Abhängigkeitspotential.
(B. Nickel, H.O. Barbe, I. Szelenyi, Arzneim.-Forsch. 40(II)8, 905-908 (1990)).
Der antinociceptive Effekt von Flupirtin läßt sich durch Naloxon nicht antagonisieren. Weiterhin zeigt Flupirtin keine Affinität zu Opiat-Rezeptoren.
(B. Nickel, A. Herz, V. Jakovlev, U. Tibes, Arzneim.-Forsch. 35(II), 1402 (1985)).

Es wurde nun gefunden, daß Flupirtin allein gegeben zu keiner Toleranzentwicklung führt. Überraschend war, daß auch in der Kombination Flupirtin und Morphin alle Anzeichen für eine Toleranzentwicklung fehlten. Dies ist nicht zu erwarten, da sich Flupirtin strukturell stark von den bekannten Morphin-Antagonisten Naloxon oder Methadon unterscheidet.

Aufgabe der Erfindung ist die Bereitstellung von verbesserten Arzneimitteln mit analgetischer Wirksamkeit, die ein stark reduziertes oder gar kein Suchtpotential mehr aufweisen.
Die in den Patentansprüchen und in der Beschreibung angegebenen Gewichtsmengen beziehen sich immer auf die freien Basen.

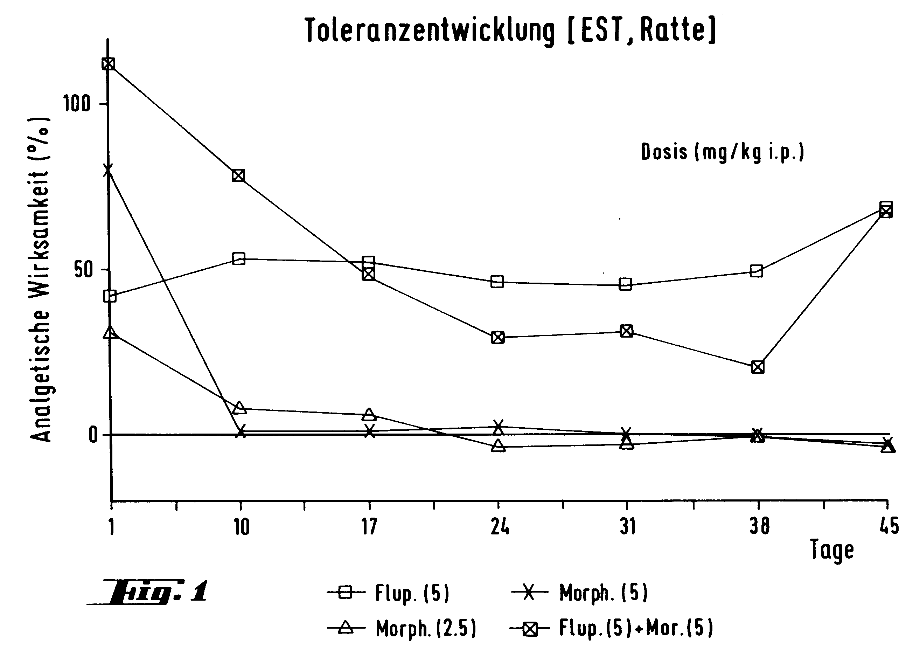

Die mehrwöchige Gabe von Flupirtin erzeugt im Tierversuch keine Toleranz. (Figur 1)
Die analgetische Wirkung bleibt über die gesamte Dauer des Versuches (45 Tage) erhalten.

Die Prüfung erfolgte am Elektroschmerztest an der Ratte (nach Blake et. al Arz. Med. exp. 9, 146 (1963))

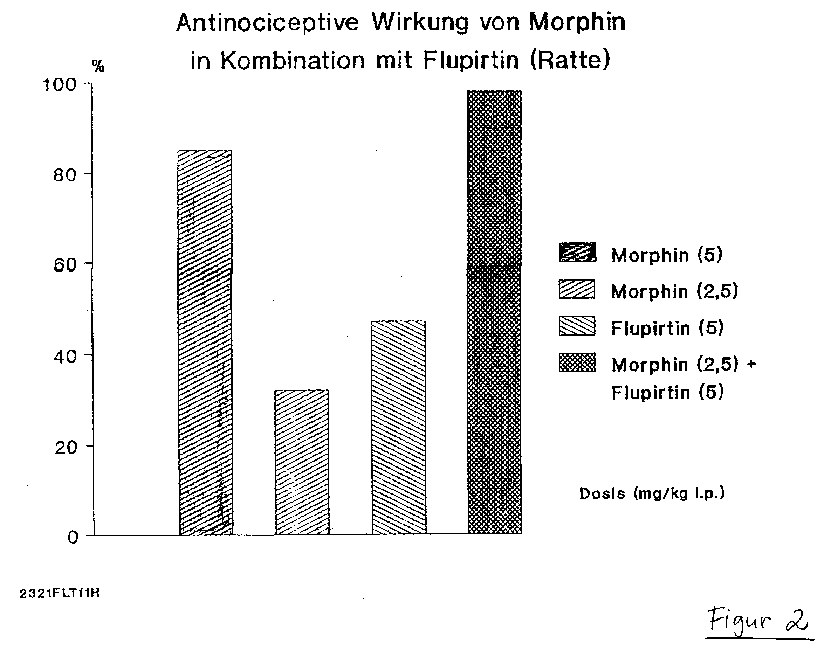

Nach der Einmalgabe von Flupirtin in Kombination mit Morphin kommt es zu einer additiven antinociceptiven Wirksamkeit. (Figur 2)
Die alleinige Gabe von Flupirtin ergibt eine antinociceptive Wirkung von 45 %, die Gabe der Kombination ergibt eine Wirkung von 100 %.

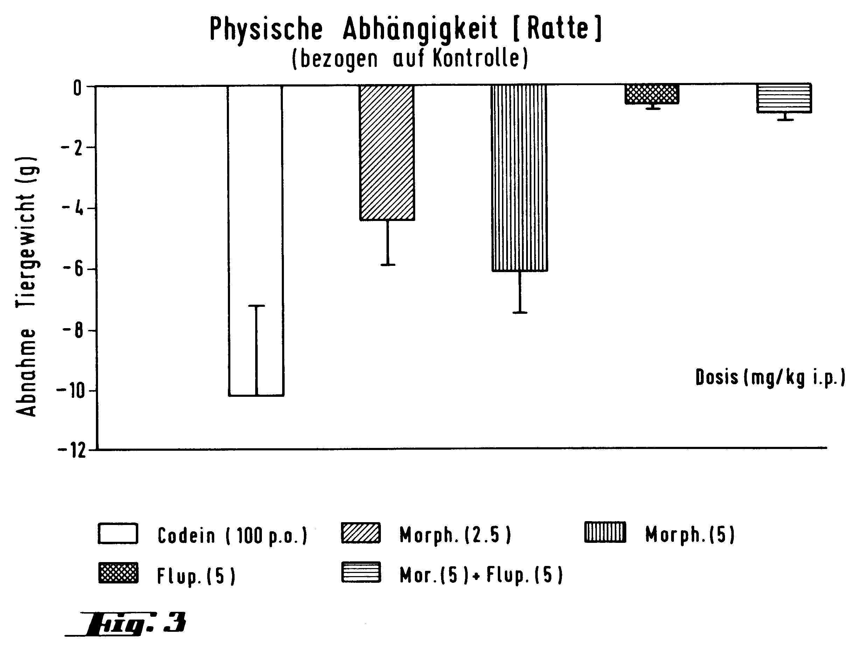

Bei der Prüfung auf physische Abhängigkeit wird das schon öfters beschriebene Abhängigkeitssymptom unter Morphin, Abnahme der Tiergewichte nach Entzug, durch Flupirtin in der Kombination mit Morphin signifikant aufgehoben (Figur 3). Flupirtin hebt beziehungsweise schwächt das physische Abhängigkeitspotential von Morphin ab.

Es ist auch anzunehmen, daß Flupirtin die durch andere Verbindungen wie die des Barbiturat-, Alkohol-, des Amphetamin-, des Cocain-, des Cannabis- oder des Halluzinogen-Typs hervorgerufenen Abhängigkeits- und Entzugssymptome aufhebt beziehungsweise deutlich abschwächt.

Die Prüfung auf das eventuelle Vorliegen einer psychischen Abhängigkeit erfolgte gemäß der Methode Hosoya, Pharmacol. Meth. Tox, 5, 515 (1979).

In derselben Langzeituntersuchung wurde das Verhalten der Tiere am Entzugstag registriert. Es zeigte sich auch in diesem Modell, daß das Verhalten der Tiere nach Entzug von Morphin (Erregung, Rearing) durch Flupirtin in der Kombination deutlich beeinflußt wird (Figur 4,5). Die deutliche Erregung beziehungsweise das Rearingverhalten der Tiere nach Morphin wird in der Kombination mit Flupirtin reduziert und ähnelt eher dem von unbehandelten Kontrolltieren. Auch wird die durch Morphin hervorgerufene Rigidität bei Tieren durch Flupirtin aufgehoben (Figur 6).

Das Arzneimittel enthält beispielsweise in einer Tablette 10 mg bis 1000 mg Flupirtin in Form eines pharmazeutisch verträglichen Salzes und 5 mg bis 500 mg Morphin in Form eines pharmazeutisch verträglichen Salzes und vorzugsweise 50 mg - 500 mg Flupirtin und 10 mg - 250 mg Morphin.

Als Salzbildner kommen im Falle des Flupirtins beispielsweise Salzsäure, Gluconsäure, Malonsäure und Maleinsäure in Frage, im Falle des Morphins kommen Mineralsäuren, wie beispielsweise Salzsäure und Schwefelsäure in Betracht.

Das erfindungsgemäße Arzneimittel kann in Form von Tabletten, Kapseln, Pellets, Granulaten, Ampullen zur intravenösen und intramuskulären Injektion, in Form von Infusionslösungen und Zäpfchen vorliegen.
Die Herstellung der Arzneimittel erfolgt in bekannter Weise, wobei die bekannten und üblichen pharmazeutischen Hilfstoffe sowie sonstige übliche Träger- und Verdünnungsstoffe verwendet werden.

Als deraritge Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953) , Seite 1 bis 39; Journal of Pharmaceutical Sciences, Band 52 (1963), Seite 918 u.ff.,
H.v.Czetsch-Lindenwald, Hilfsstoffe für Pharmazie und angrenzende Gebiete; Pharm. Ind., Heft 2, 1961, Seite 72 u.ff.; Dr. H. P. Fiedler, Lexikon der Hilfsstoffe für Pharmazie, Kosmetik und angrenzende Gebiete, 2. Auflage, Editio Cantor, Aulendorf in Württemberg 1981 und Pharmazeutische Technologie (Hrgs. Fuchs, Sucker, Speiser, Georg Thieme Verlag, 2. Auflage (1991).

### Figurenbeschreibung

Figur 1 beschreibt die Toleranzentwicklung über 45 Tage der Kombination im Vergleich zu den Einzelstoffen.

Figur 2 zeigt die antinociaptive Wirkung der Kombination im Vergleich zu den Einzelstoffen.

Figur 3 stellt die Ergebnisse eines Versuchs zur psychischen Abhängigkeit dar.

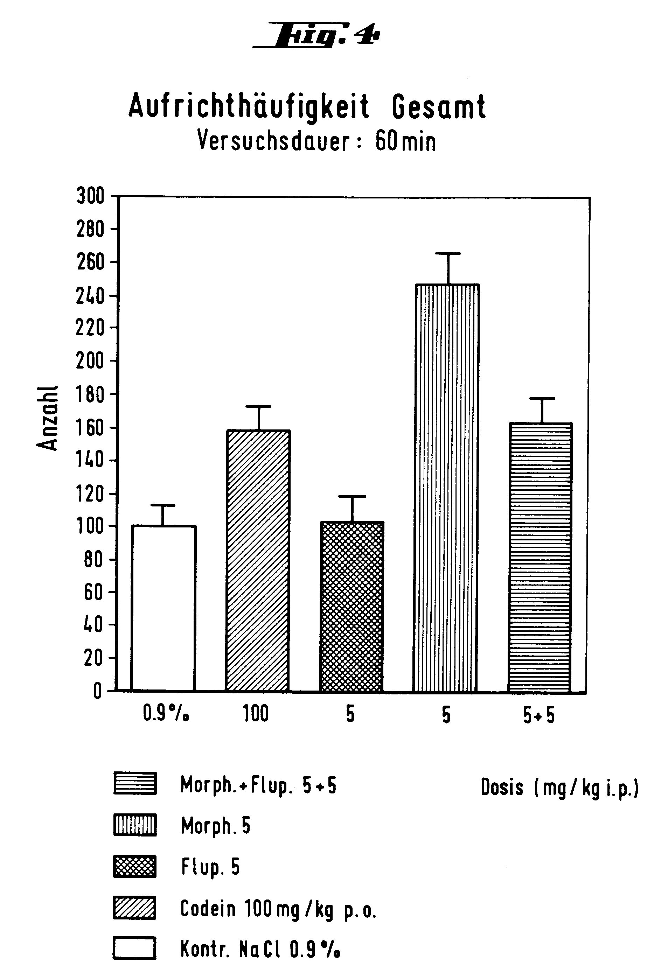

Figur 4 zeigt anhand der Aufrichthäufigkeit von Ratten die erregende Wirkung von Morphin alleine und die nicht erregende Wirkung der erfindungsgemäßen Kombination.

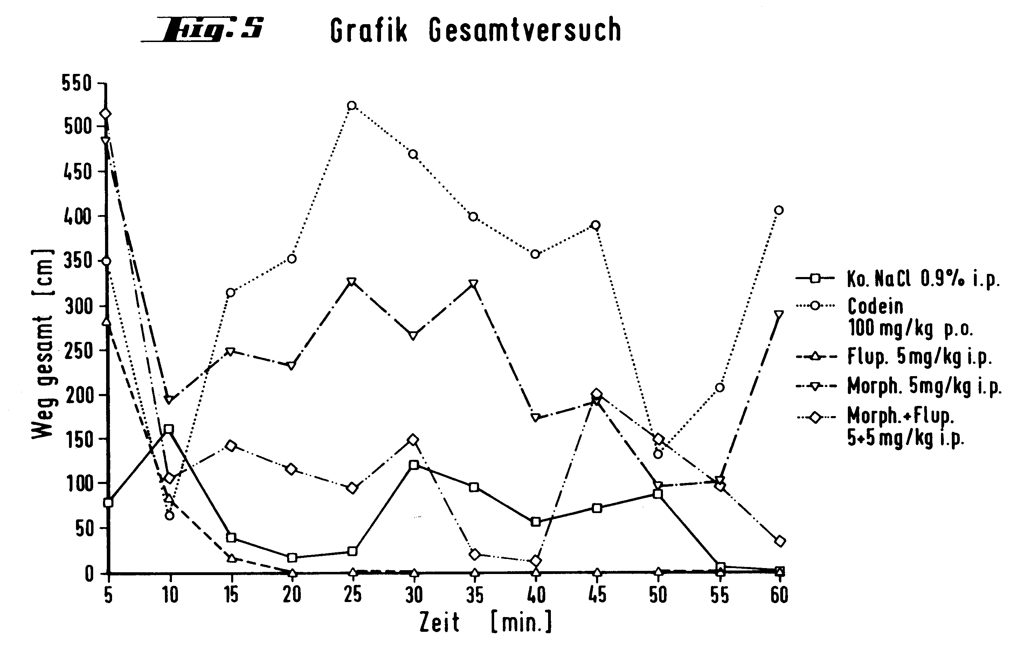

Figur 5 zeigt einen ähnlichen Versuch wie Figur 4: hier wird als Maß für die Erregung der Tiere die Wegstrecke gemessen.

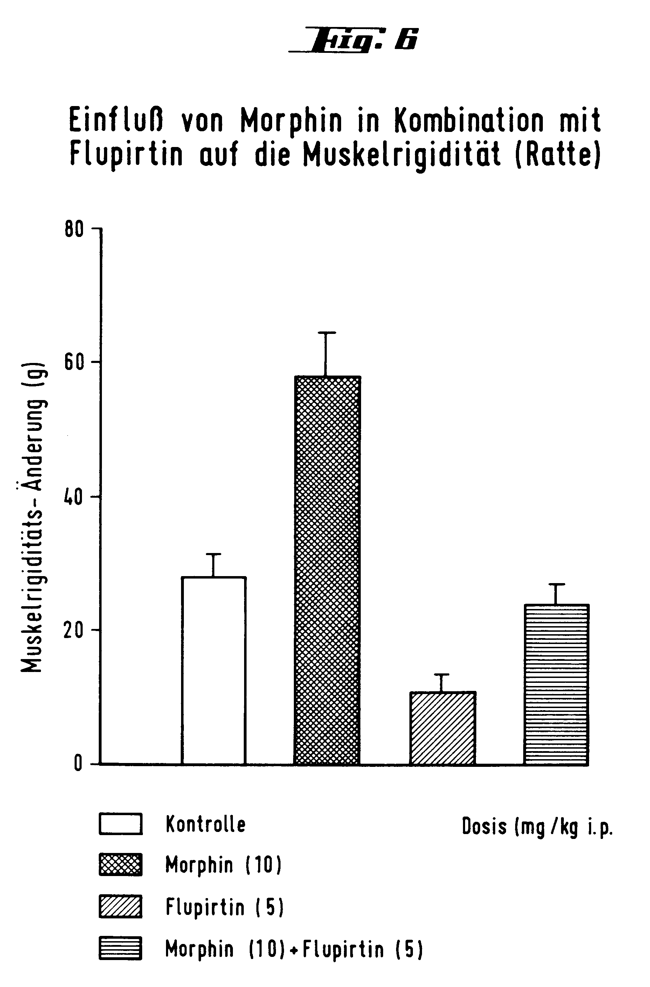

Figur 6 zeigt den Einfluß der Einzelsubstanzen auf die Muskelrelaxation im Vergleich zur erfindungsgemäßen Kombination.

## Patentansprüche

1. Arzneimittel,
**dadurch gekennzeichnet**,
daß es aus Flupirtin oder einem pharmazeutisch annehmbaren Salz hiervon, in der Menge von 10 mg bis 1000 mg bezogen auf die Base, und einem pharmazeutisch verträglichen Salz des Morphins in der Menge von 5 mg bis 500 mg, bezogen auf die Base, besteht.

2. Verfahren zur Herstellung eines Arzneimittels, gemäß Anspruch 1,
**dadurch gekennzeichnet**,
daß man Flupirtin oder ein pharmazeutisch verwendbares Salz in der Menge von 10 mg bis 1000 mg mit Morphin oder ein pharmazeutisch verwendbares Salz hiervon in der Menge von 5 mg bis 500 mg mit konventionellen Trägerstoffen und Hilfsstoffen zu einem Arzneimittel verarbeitet.

3. Verwendung eines Gemisches aus Flupirtin oder ein pharmazeutisch annehmbares Salz hiervon, in der Menge von 10 mg bis 1000 mg bezogen auf die Base, und einem pharmazeutisch verträglichen Salz des Morphins in der Menge von 5 mg bis 500 mg, bezogen auf die Base, zur Herstellung eines Arzneimittels.

## Claims

1. Pharmaceutical,
characterised in that
it comprises flupirtine or a pharmaceutically acceptable salt thereof, in a quantity of from 10 mg to 1000 mg, with reference the base, and a pharmaceutically tolerable salt of morphine in a quantity of from 5 mg to 500 mg, with reference to the base.

2. Method for the preparation of a pharmaceutical according to claim 1,
characterised in that
flupirtine or a pharmaceutically usable salt in a quantity of from 10 mg to 1000 mg together with morphine or a pharmaceutically usable salt thereof in a quantity of from 5 mg to 500 mg together with conventional carriers and auxiliary substances are processed into a pharmaceutical.

3. Use of a mixture of flupirtine or a pharmaceutically acceptable salt thereof, in a quantity of from 10 mg to 1000 mg, referred to the base, and a pharmaceutically tolerable salt of morphine in a quantity of from 5 mg to 500 mg, with reference to the base, for the preparation of a pharmaceutical.

## Revendications

1. Médicament caractérisé en ce qu'il consiste en la flupirtine ou un sel pharmaceutiquement acceptable de celle-ci, en quantité allant de 10 à 1000 mg rapporté à la base, et en un sel pharmaceutiquement compatible de morphine en quantité allant de 5 à 500 mg rapporté à la base.

2. Procédé d'obtention d'un médicament selon la revendication 1, caractérisé en ce que l'on transforme la flupirtine ou un de ses sels utilisables pharmaceutiquement en une quantité allant de 10 à 1000 mg avec de la morphine ou un sel pharmaceutiquement utilisable de celle-ci en une quantité allant de 5 à 500 mg avec des substances support et des adjuvants conventionnels, en un médicament.

3. Utilisation d'un mélange à base de flupirtine ou d'un de ses sels pharmaceutiquement acceptable en quantité allant de 10 à 1000 mg rapporté à la base, et d'un sel pharmaceutiquement compatible de morphine en une quantité allant de 5 à 500 mg rapporté à la base, pour l'obtention d'un médicament.
